# EUROPÄISCHE PATENTSCHRIFT

(11) **EP 2 273 957 B1**
(45) Veröffentlichungstag und Bekanntmachung des Hinweises auf die Patenterteilung: **20.06.2012**
(21) Anmeldenummer: 09737748.5
(22) Anmeldetag: 29.04.2009
(51) Int. Cl.: A61F 5/56

(54) **ANTISCHNARCHVORRICHTUNG**
ANTI-SNORING DEVICE
DISPOSITIF ANTI-RONFLEMENTS

(30) Priorität: 02.05.2008 DE 202008006076 U
(43) Veröffentlichungstag der Anmeldung: 19.01.2011
(73) Patentinhaber: Li, Wu, 81677 München (DE)
(72) Erfinder: Li, Wu, 81677 München (DE)
(74) Vertreter: Rösler, Uwe
(86) Internationale Anmeldenummer: PCT/DE2009/000614
(87) Internationale Veröffentlichungsnummer: WO 2009/132640

(56) Entgegenhaltungen:
- DE-U1- 9 409 999
- FR-A- 2 863 863
- GB-A- 874 480
- US-A- 5 752 822
- US-A- 5 988 170

## Beschreibung

### Technisches Gebiet

Die Erfindung bezieht sich auf eine Antischnarchvorrichtung zur Positionierung innerhalb der Mundhöhle einer Person, mit einem Verdrängungskörper, dessen Form und Größe eine Platzierung innerhalb der Mundhöhle zwischen Zungenunterseite und Mundbodenmuskulatur gestattet und die Zunge zur Mundhöhle hin anhebt, wobei der Verdrängungsköper mit einem bissschienenartig ausgebildeten Element in Wirkverbindung steht, das zwischen den Zähnen des Ober- und Unterkiefers platzierbar ist und wenigstens eine Durchgangsöffnung zu Belüftung der Mundhöhle mit Atemluft vorsieht.

Das Schlafatmungsgeräuch, das Schnarchen, wird insbesondere für Personen in der Umgebung des Schnarchers als sehr störend empfunden. Darüber hinaus ist Schnarchen für Schnarcher sogar gesundheitsgefährdend, wenn z. B. eine obstruktive Schlafapnoe vorliegt. In der Regel entsteht schnarchen durch den schwachen Muskeltonus der Halsmuskulatur während des Schlafes. Es kommt dabei zu Engpässen der Atemwege bis hin zum Verschluss (Apnoe). Dies führt zu einem erhöhten Luftstrom und zur Geräuschbildung der in Bewegung geratenen erschlafften Strukturen. Ohnehin nimmt auf natürliche Weise die Muskelspannung bei älteren Menschen im Rachenraum ab, weshalb dieser Personenkreis häufiger als jüngere Menschen schnarcht.

### Stand der Technik

Zur Verhinderung des Schnarchens ist eine ganze Reihe von Geräten, Hilfsmittel, Atemmasken, einschließlich operativer Eingriffe bekannt.

Operationen dienen in der Regel der Entfernung von die Atmung behindernden Strukturen wie vergrößerte Mandeln, Polypen usw.. Die Erfolgsrate, das Schnarchen betreffend, wird jedoch sehr unterschiedlich mit 20-40% angegeben. Mehreren Berichten zufolge ist die Rückfallquote sehr hoch.

Die Atemmaske dient zur Überdruckbeatmung und hält dadurch die Atemwege offen. Da sie sehr aufwendig ist und eine für den Schnarcher unbequeme Einrichtung darstellt, ist sie hauptsächlich in schweren Fällen von Apnoe indiziert.

Intraorale Geräte werden nachts getragen und verlagern in der Regel den Unterkiefer nach vorn, um eine Öffnung des pharyngealen Anteils der Atemwege zu erreichen. Mit dem Unterkiefer wird auch der Zungengrund mit nach vorne genommen, wodurch die Öffnung der Atemwege möglich wird. Erfolge kann man mit diesen Geräten erzielen, infolge ihrer Konstruktion und Bauart werden sie vom Träger jedoch meist als sehr lästig empfunden.

Bekannte orale Vorrichtungen gegen Schnarchen sind repräsentativ für eine Vielzahl bekannter gattungsgemäßer Vorrichtungen aus den folgenden Druckschriften zu entnehmen:

Die DE 40 26 602 C1 beschreibt eine Vorrichtung zum Verhindern des Schnarchens, mit einem Protheseteil, das an den Zähnen befestigt wird und an das ein Bügel über einen Federmechanismus derart angebracht ist, dass einerseits die Zunge vom hinteren Gaumensegel wegdrückt und andererseits die Zunge beim Schluckvorgang an den Gaumen drücke kann.

Die EP 0 312 368 B1 beschreibt eine modifizierte Bissschiene als Antischnarchvorrichtung, auf die die Zähne des Oberkiefers aufbeißen, wobei eine untere Schubkulisse der Bissschiene in Richtung der Mundhöhlenöffnung gegen die Innenseiten der Zähne des Unterkiefers drückt, wodurch der Unterkiefer nach vom geschoben und dabei der Abstand zwischen der Zunge und dem weichen Gaumen vergrößert wird.

Die EP 0 254 918 A1 beschreibt eine Ober- und Unterkieferschiene mit eingelagerten Magneten, die durch gegenseitige magnetische Abstoßung beide Kiefer in einem bestimmen Abstand halten sollen. Die DP 27 04 709 A1 stellt einen regelrechten Mundsperrer dar.

Die EP 0 599 445 A1 beschreibt ein Gerät mit fester Fixierung des Oberkiefers und loser Fixierung des Unterkiefers. Die orale Seite des Gerätes ist so ausgebildet, dass sich die Zunge saugend einlagern kann.

Die WO 92/05752 beschreibt ein Gerät mit Gaumenplatte und Mundbodenplatte, das gleichzeitig eine Anteriorisierung des Unterkiefers und den Zwang zur Nasenatmung bewirken soll, dabei werden zwei Varianten beschrieben: einmal mit Fixierung des Gerätes an den Zähnen, und zum andern sitzt das Gerät frei im Mund.

Die WO 92/09249 beschreibt ein Gerät um Zungenpressen, Zähneknirschen und Schnarchen zu verhindern. Zur Zunge und zu den Zähnen angepasst, soll die Vorrichtung durch Saugen die Zunge vorne halten.

Die US Patentschrift US 5,092,346 beschreibt ein Gerät für den Oberkiefer mit einer Rampe für den Unterkiefer, um diesen zu anteriorisieren, sowie einer Atemöffnung zwischen Oberkieferteil und Unterkieferrampe.

Die DE 23 20 501 C3 beschreibt ein Gerät, das ähnlich einem kieferorthopädischen Aktivator konstruiert ist, mit einem zusätzlichen labialen und verstellbaren Lippenschild. Dabei soll durch Muskelaktivität und entsprechende Schliffführungen im Gerät für die Zähne der Unterkiefer anteriorisiert werden. Doch gerade im Schlafzustand erschlafft die Muskulatur, und der Muskeltonus ist herabgesetzt bzw. aufgehoben.

Die DE G 94 09 999.5 U1 beschreibt ein Gerät zur Verhinderung der durch Schnarchen verursachten Schlafapnoe, das einen innerhalb der Mundhöhle einer Person platzierbaren Verdrängungskörper vorsieht, der zwischen Zungenunterseite und Mundbodenmuskulatur zu liegen kommt und die Zunge zur Mundhöhle hin anhebt. Zudem weist der Verdrängungsköper ein bissschienenartig ausgebildetes Element auf, das zwischen den Zähnen des Ober- und Unterkiefers platzierbar ist und wenigstens eine Durchgangsöffnung zu Belüftung der Mundhöhle mit Atemluft vorsieht. Eine vergleichbare Anordnung ist der US 5,752822 zu entnehmen

Der Hauptnachteil der bekannten Geräte liegt darin, dass die Vorrichtungen infolge ihrer voluminösen Ausdehnung, ihrer Zwänge durch eine Bisssperrung, ihres Druckes auf die Zunge oder weiche Gaumenteile, der eingeschränkten Bewegungsfreiheit des Unterkiefers und der Zunge ausgesprochen unbequem beim Tragen sind. Durch das Gefühl einen enorm störenden Fremdkörpers im Mund wird der Schnarcher am Einschlafen gehindert, was in der Regel zur Nichtbenutzung der Geräte führt..

### Darstellung der Erfindung

Es besteht die Aufgabe eine Antischnarchvorrichtung anzugeben, die die vorstehenden Nachteile zu beseitigen vermag. Insbesondere gilt es eine oral zu tragende Antischnarchvorrichtung zu schaffen, die dem Träger die nötige Schlaftiefe und sein Wohlbefinden gibt, insbesondere durch die geringe Ausdehnung im Kieferbereich und hohen Tragekomfort der Antischnarchvorrichtung.

Die Lösung der der Erfindung zugrunde liegenden Aufgabe ist im Anspruch 1 angegeben. Den Erfindungsgedanken vorteilhaft weiterbildende Merkmale sind Gegenstand der Unteransprüche sowie der weiteren Beschreibung insbesondere unter Bezugnahme auf die Ausführungsbeispiele zu entnehmen.

Lösungsgemäß ist eine Antischnarchvorrichtung zur Positionierung innerhalb der Mundhöhle einer Person mit einem Verdrängungskörper, dessen Form und Größe eine Platzierung innerhalb der Mundhöhle zwischen Zungenunterseite und Mundbodenmuskulatur gestattet und der die Zunge zur Mundhöhle hin anhebt und mit einem bissschienenartig ausgebildeten Element in Wirkverbindung steht, das zwischen den Zähnen des Ober- und Unterkiefers platzierbar ist und wenigstens eine Durchgangsöffnung zu Belüftung der Mundhöhle mit Atemluft vorsieht, derart ausgebildet, dass der Verdrängungskörper kugelartig ausgebildet und hinsichtlich Form und Größe weitgehend an die geometrischen Gegebenheiten der intraoralen Anatomie einer Person angepasst, so dass bei oralem Einbringen der Vorrichtung der kugelförmige Verdrängungskörper unter die Zunge zwischen Zungenunterseite und Mundbodenmuskulatur zu liegen kommt, wobei die Zunge zum Gaumen hin gedrückt wird ohne dabei einen unangenehmen oralen Verdrängungseindruck bei der jeweiligen Person hervorzurufen. So sollte der Kugeldurchmesser des kugelförmigen Verdrängungskörpers zwischen 1,5 und 2 cm liegen. Durch die Anordnung des kugelartigen Elements an der unteren Fläche der Zunge wird die erschlaffte Zungenmuskulatur unterstützt und in Richtung Zähne gezogen und so der Atemweg geöffnet.

Alternativ ist der Verdrängungskörper lösungsgemäß in Form zweier kugelförmiger Elemente ausgebildet, deren Kugelgeometrien sich lokal teilweise durchdringen. Ein derartig ausgebildeter Verdrängungskörper wird oral derart positioniert, dass je ein kugelförmiges Element rechts und links vom Zungenbändchen zu liegen kommt.

Der Verdrängungskörper ist vorzugsweise fest über einen Verbindungsabschnitt mit dem bissschienenartigen Element verbunden, das über eine gekrümmt länglich, strangartig ausgebildete Form verfügt, die eine obere und untere Bissfläche aufweist, auf die jeweils die vorderen Zähne, vorzugsweise jene von den Eckzähnen seitlich begrenzten Zähne des Ober- und Unterkiefers beissen können.

Das bissschienenartige Element ist zu Zwecken einer ungehinderten Atemluftversorgung für die Person mit wenigstens einer, vorzugsweise vieler Durchgangsöffnungen durchsetzt. Um den Tragekomfort der lösungsgemäßen Vorrichtung für die Person so angenehm wie möglich zu gestalten, sollte das bissschienenartige Element eine während des Bisses wahrnehmbare Dicke von 2 cm nicht überschreiten.

In einer weitere Ausgestaltungsform der lösungsgemäßen Vorrichtung bietet es sich an das bissschienenartig ausgebildete Element oder den Verdrängungskörper über eine verstellbare Zugvorrichtung, die an den Ohren gehalten wird, zu verbinden, so dass die Zunge im Schlaf in Richtung Gaumen in eindeutiger Stellung gedrückt wird. Die Zugvorrichtung sollte aus einem schnurähnlichen, flexiblen Material bestehen und Mittel zur Längenveränderung aufweisen.

Wie bereist erwähnt sind die Form und Größe der kugelartigen Elemente sowie des bissschienenartig ausgebildeten Elements wichtig für den Tragekomfort und müssen an die jeweilige Ausbildung der Atemwege der Person angepasst werden. Dies gilt auch für die Ausbildung der Zugvorrichtung, die Mittel zum Einhängen an den Ohren aufweist. Sämtliche verwendeten Materialien zur Realisierung der lösungsgemäßen Vorrichtung müssen widerstandsfähig gegen Mundflüssigkeit und physiologisch unbedenklich sein.

Zur Unterstützung der oral getragenen Antischnarchvorrichtung ist es in spezifischen Fällen zweckmäßig zum Offenhalten der Nasenflügel elastische Formstöpsel mit Durchgangsöffnung einzufügen, die durch die elastische Spannung im Nasenflügel gehalten werden. Durch diese Maßnahme wird sichergestellt, dass der Atemweg über die Nasenhöhle offen gehalten wird und die Zuluft geregelt wird.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigen:
- Fig. 1: Illustration freier Atemwege,
- Fig. 2: Illustration verschlossener Atemwege bei obstruktiven Apnoe,
- Fig. 3: Schematische Darstellung der Funktionsweise der lösungsgemäßen Antischnarchvorrichtung,
- Fig. 4 a-e: Mehrseitendarstellungen eines ersten Ausführungsbeispiels und
- Fig. 5 a-c: Mehrseitendarstellungen eines zweiten Ausführungsbeispiels.

### Wege zur Ausführung der Erfindung, gewerbliche Verwendbarkeit

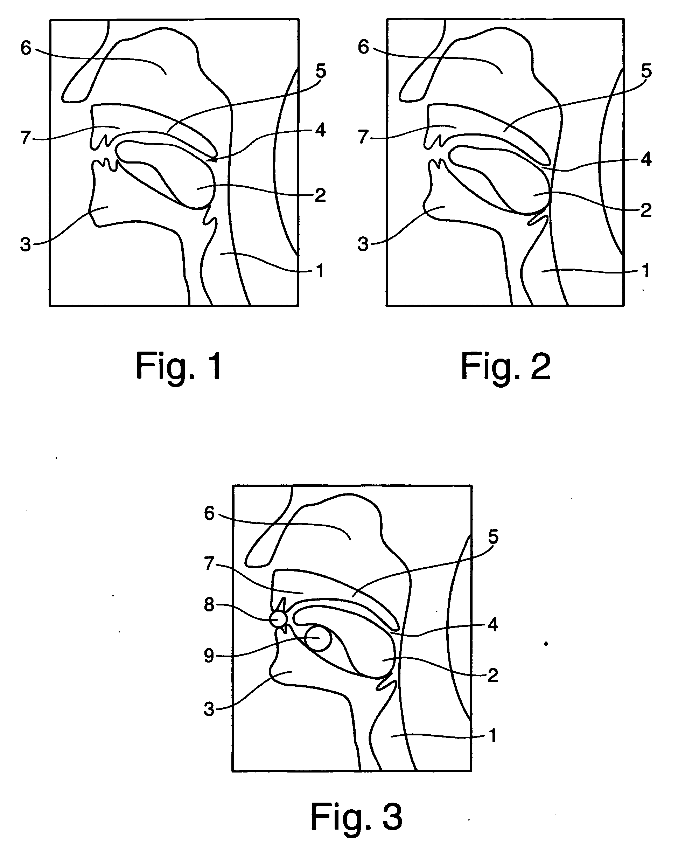

Fig. 1 zeigt eine schematische Darstellung eines Schnittes durch die menschlichen Atemwege. Die Luft wird über die Nasenhöhle 6 und die Mundhöhle 4 in die Luftröhre 1 geleitet. Wichtig hierbei ist, dass der Zugang zur Luftröhre 1 nicht durch die Zunge 2 oder das Gaumensegel ganz oder teilweise eingeengt wird. Auch muss der Zugang der Luft zwischen Zunge 2 und weichem Gaumen 5 aufrechterhalten werden. Weiterhin ist in Fig. 1 der Unterkiefer 3 dargestellt, der sich bei Schnarchern zur Luftröhre hin verschiebt.

In Fig. 2 ist die Situation dargestellt (schematisch), wenn die Atemwege verschlossen sind, was zu schnarchen führt, aber auch ein teilweiser Verschluss kann schnarchen auslösen (nicht dargestellt). In dieser Situation schiebt sich die Zunge 2 aufgrund der sich erschlaffenden Zungenmuskulatur nach hinten und verschließt zumindest zeitweise den Luftweg durch die Luftröhre 1.

Diese Situation vermag die lösungsgemäße Vorrichtung zu vermeiden helfen. Durch die erfindungsgemäße Vorrichtung wird die beim Schlafen erschlaffte Zungenmuskulatur zur Mundhöhle hin angehoben, was bewirkt, dass die Luftröhre nicht gänzlich verschlossen wird. Die untere Fläche der Zunge ist mit ihrem mittleren Teil an dem Boden der Mundhöhle angewachsen und vom durch eine Falte der Mundschleimhaut, das Zungenbändchen, so angeheftet, dass nur die Spitze und die Seitenränder frei sind.

Das kugelartige Element 9 wird, wie Fig. 3 zeigt, an der unteren Fläche der Zunge 2 im mittleren Bereich neben dem Zungenbändchen platziert. Verfügt die Antischnarchvorrichtung über zwei kugelartige Elemente 9, so wird je ein kugelartiges Element 9 rechts und links vom Zungenbändchen platziert. Die kugelartigen Elemente haben einen Durchmesser von 1,5 bis 2,0 cm, je nach Ausbildung der Zunge des Trägers.

Die kugelartigen Elemente 9 sind über ein Verbindungsmittel mit einem bissschienenartigen Element 8 verbunden, das zwischen den Zähnen zum Liegen kommt, wenn die kugelartigen Elemente 9 in ihre vorgesehene Stellung gebracht wurden. Das bissschienenartige Element 8 verfügt über mindestens eine Querbohrung mit einem Durchmesser von 2,5 bis 3,5 mm, so dass Luft zum Atmen durchströmen kann.

Bei Bedarf können auch zwei und mehrere Bohrungen vorgesehen werden. Das bissschienenartige Element 8 dient zusätzlich dazu, die Mundhöhle 4 zu vergrößern, so dass die gesamte Mundhöhle mehr Luftraum hat.

Der orale Teil der Antischnarchvorrichtung besteht somit aus zwei miteinander verbundenen Teilen: dem kugelartigen Element 9 das unter der Zunge liegt und dem bissschienenartigen Element 8, das zwischen den Zähnen des Ober- und Unterkiefers liegt.

Da das bissschienenartige Element 8, wie oben beschrieben, mehr Luftraum erzeugt, ist in bestimmten Fällen auch Sorge zu tragen, dass die Nasenöffnung derart reguliert wird, dass nicht zu viel Atemluft eingeatmet wird. Die gesamte Atemluft wird hierbei in Balance gebracht. Zu diesem Zweck dienen elastische Formkörper mit Durchgangsöffnung die unter Spannung in die Nasenlöcher eingesetzt werden. Diese Formkörper haben eine Länge von ca. 2 cm-3 cm und einen Durchmesser von 1,0 bis 1,5 cm. Im Ruhezustand sind die Formkörper auf der Unterseite eben und die Oberseite ist gewölbt. Vor dem Einsatz wird der Formkörper etwas zusammen gerollt, so dass er in die Nasenöffnung einschiebbar ist. Verwendet man die Formkörper paarweise so ist es zweckmäßig diese zu verbinden. Das Verbindungselement kann auch als Spannelement dienen, das die Formkörper gegen die Naseninnenwand drückt.

Optional können die kugelartigen Elemente 9 über eine nicht dargestellte verstellbare Zugvorrichtung miteinander verbunden sein; je ein Ende der Zugvorrichtung ist mit einer Öse ausgestattet, die zum Halt über je eine Ohrmuschel gezogen wird. Die Ösen können je nach Bedarf vergrößert werden. Im Allgemeinen besteht die Zugvorrichtung aus einem elastischen Band, das widerstandsfähig gegen Mundflüssigkeit und physiologisch unbedenklich sein muss. Das kugelartige Element 9 oder die beiden kugelartigen Elemente 9 sind auf die Zugvorrichtung aufgefädelt und notfalls darauf fixiert. Aus diesem Grund weisen die kugelartigen Elemente 9 eine zentrale Bohrung auf. Mit Hilfe der am Ohr fixierten Zugvorrichtung gelingt es den Anpressdruck des kugelartigen Elementes 9 von unten an die Zunge zu erhöhen.

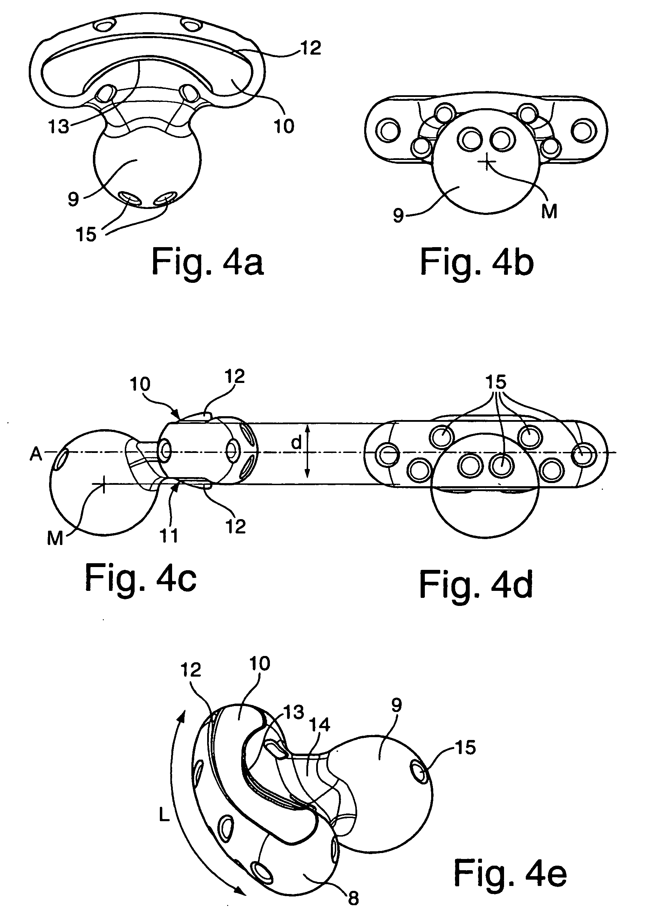

In den Bilddarstellungen gemäß den Figuren 4a bis e sind perspektivische Ansichten einer bevorzugt ausgebildeten Antischnarchvorrichtung dargestellt, auf die im Folgenden gesamtheitlich Bezug genommen wird.

Über einen Verbindungsabschnitt 14 ist der kugelförmig ausgebildete Verdrängungskörper 9 mit der Konkavseite des länglich gekrümmten, bissschienenartig ausgebildeten Elementes 8 fest verbunden. Die in den Figuren 4 a bis e illustrierte Antischnarchvorrichtung ist einstückig ausgebildet und besteht aus einem biokompatiblen, bissfesten Kunststoff.

Das bissschienenartig ausgebildete Element 8 weist eine obere und untere Bissfläche 10 und 11 auf, auf die insbesondere die vorderen Zähne des Ober- und Unterkiefers, die vorzugsweise jeweils von den Eckzähnen seitlich begrenzt sind, aufbeißen. Dass bissschienenartig ausgebildete Element 8 weist somit eine gekrümmte Länge L typischerweise zwischen 3 und 4 cm auf, je nach den oralanatomischen Verhältnissen einer Person. Zur Vermeidung eines Überbisses sowie der Unterstützung einer intraoralen selbstzentrierenden Positionierung der Antischnarchvorrichtung innerhalb der Mundhöhle sind beide Bissflächen 10, 11 zumindest von einer vorderen Begrenzungskante 12 begrenzt, die sich vorzugsweise senkrecht über die jeweiligen Bissflächen 10, 11 erheben. Nicht notwendigerweise aber in vorteilhafter Ausbildung sind die Bissflächen 10, 11 auch rückwärtig von einer entsprechenden Begrenzungskante 13 begrenzt.

Die sog. Dicke des bissschienenartig ausgebildeten Elementes 8 durch die die Kieferöffnung vorgegeben wird, ist durch den orthogonalen Abstand d zwischen beiden Begrenzungsflächen 10, 11 vorgegeben, der wenigstens 5 mm trägt, jedoch 2 cm nicht überschreiten sollte.

Zur ausreichenden Versorgung der Person mit Atemluft durch die Mundhöhle hindurch, sieht das bissschienenartig ausgebildete Element 8 eine Vielzahl von kanalartig ausgebildeten Durchgangsöffnungen 15 vor, typischerweise mit Öffnungsdurchmesser zwischen 2,5 und 3,5 mm. Auch die Anzahl und Anordnung der Durchgangsöffnungen 15 sind in Abhängigkeit des Atmungsverhaltens einer jeweiligen Person anpassbar.

Aufgrund der an die natürliche Bissfläche der Zähne des Ober- und Unterkiefer länglich gekrümmt angepassten Formgebung des bissschienenartig ausgebildeten Elementes 8 weist dieses eine konkave Innenseite auf, an der über einen Verbindungsabschnitt 14 der kugelförmig ausgebildete Verdrängungskörper 9 angebracht ist. Die Länge des den kugelförmigen Verdrängungskörper 9 von dem bissschienenartig ausgebildeten Element 8 beabstandeten Verbindungsabschnittes 14 ist so gewählt, dass bei oraler Positionierung der Antischnarchvorrichtung der kugelförmige Verdrängungskörper 9 zwischen Zunge und Mundbodenmuskulatur unmittelbar angrenzend zum Zungenbändchen zu liegen kommt. Über den Verbindungsabschnitt 14 ist der kugelförmige Verdrängungskörper 9 derart relativ zum bissschienenartig ausgebildeten Element 8 angebracht, so dass der kugelförmige Verdrängungskörper 9 in dem gegenüber den Zähnen des Unterkiefers vertieft abgesenkten Bereich der Mundbodenmuskulatur zu liegen kommt. Aus diesem Grund befindet sich der Kugelmittelpunkt M des kugelförmigen Verdrängungskörpers 9 unterhalb einer Mittenebene A, die sich mittig zwischen beiden Bissflächen 10, 11 erstreckt. Im dargestellten Ausführungsbeispiel befindet sich die räumliche Lage des Kugelmittelpunktes M in der Ebene der unteren Bissfläche 11 des bissschienenartig ausgebildeten Elementes 8 (siehe Fig. 4c).

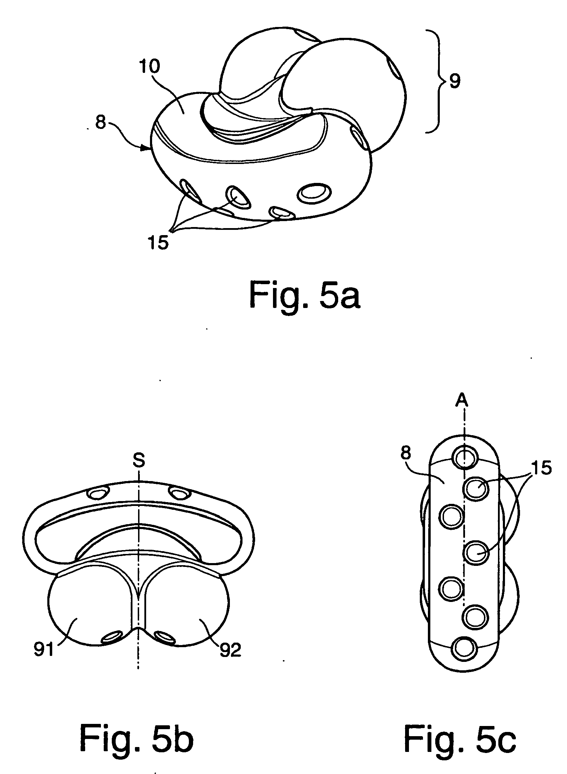

Ein weiteres Ausführungsbeispiel ist in den Figuren 5a bis c illustriert, die ebenfalls räumliche Ansichten aus unterschiedlichen Blickwinkeln auf ein und dasselbe Ausführungsbeispiel zeigen. Im Unterschied zum vorstehen erläuterten Ausführungsbeispiel weist das in Figur 5 illustrierte Ausführungsbeispiel zwei kugelartige Elemente 91, 92 auf, die gemeinsam den Verdrängungskörper 9 darstellen. Das bissschienenartig ausgebildete Element 8 ist weitgehend identisch zu jenem im Ausführungsbeispiel gemäß der Figur 4 ausgebildet. Die kugelartigen Elemente 91, 92 schmiegen sich symmetrisch zu der in Figur 5b eingezeichneten Symmetrieachse S nahezu nahtlos an das bissschienenartig ausgebildete Element 8 an. Die kugelartigen Elemente 91, 92 werden bei oraler Positionierung jeweils links und rechts zum Zungenbändchen platziert, so dass das Zungenbändchen in etwa längs der in Figur 5b eingezeichneten Symmetrieachse S zu liegen kommt. Auch im Falle der Antischnarchvorrichtung gemäß Figur 5 sind die kugelartigen Elemente 91, 92 gegenüber der vorstehend erläuterten Mittenebene A leicht abgesenkt (siehe Figur 5c), so dass die kugelartigen Elemente 91, 92 innerhalb des Bereiches der Mundbodenmuskulatur zu liegen kommen.

Sämtliche Oberflächen der lösungsgemäß ausgebildeten Antischnarchvorrichtung sind glatt und abgerundet ausgebildet, so dass keinerlei Verletzungsgefahr innerhalb der Mundhöhle besteht. Um den Tragekomfort für eine Person zu verbessern, sollte der bissfeste und biokompatible Kunststoff möglichst leichtgewichtig ausgebildet sein. Im Übrigen ermöglicht die lösungsgemäße Antischnarchvorrichtung eine kostengünstige Herstellung bspw. mittels Spritzgussverfahren.

### Bezugszeichenliste

- 1: Luftröhre
- 2: Zunge
- 3: Unterkiefer
- 4: Mundhöhle
- 5: Weicher Gaumen
- 6: Nasenhöhle
- 7: Oberkiefer
- 8: Bissschienenartig ausgebildetes Element
- 9: Verdrängungskörper, kugelartiges Element
- 91, 92: Kugelartiges Element
- 10: Obere Bissflächen
- 11: Untere Bissfläche
- 12: Vordere Begrenzungskante
- 13: Hintere Begrenzungskante
- 14: Verbindungsabschnitt
- 15: Durchgangsöffnung
- M: Kugelmittelpunkt
- L: Gekrümmte Länge des bissschienenartig ausgebildeten Elementes
- A: Mittenebene
- d: Abstand zwischen der Bissflächen
- S: Symmetrieachse

## Patentansprüche

1. Antischnarchvorrichtung zur Positionierung innerhalb der Mundhöhle einer Person, mit einem Verdrängungskörper (9), dessen Form und Größe eine Platzierung innerhalb der Mundhöhle (4) zwischen Zungenunterseite und Mundbodenmuskulatur gestattet und die Zunge (2) zur Mundhöhle hin anhebt, wobei der Verdrängungsköper (9) mit einem bissschienenartig ausgebildeten Element (8) in Wirkverbindung steht, das zwischen den Zähnen des Ober- und Unterkiefers (7, 3) platzierbar ist und wenigstens eine Durchgangsöffnung (15) zu Belüftung der Mundhöhle mit Atemluft vorsieht,
**dadurch gekennzeichnet, dass** der Verdrängungskörper (9) kugelförmig ausgebildet ist, oder dass der Verdrängungskörper (9) zwei gleichförmig kugelartig ausgebildete Elemente (91, 92) aufweist, deren Kugelformen sich lokal teilweise durchdringen.

2. Antischnarchvorrichtung nach Anspruch1,
**dadurch gekennzeichnet, dass** der kugelförmige Verdrängungskörper (9) oder die kugelartigen Elemente (91, 92) jeweils einen Durchmesser zwischen 1,5 und 2,0 cm besitzen.

3. Antischnarchvorrichtung nach Anspruch 1 oder 2,
**dadurch gekennzeichnet, dass** der Verdrängungskörper (9) und das bissschienenartige Element (8) über einen Verbindungsabschnitt (14) fest verbunden sind.

4. Antischnarchvorrichtung nach einem der Ansprüche 1 bis 3,
**dadurch gekennzeichnet, dass** das bissschnienenartig ausgebildete Element (8) länglich gekrümmt ausgebildet ist, mit einer oberen und unteren Bissfläche (10, 11), die einen orthogonal zu beiden Bissflächen orientierten Abstand von wenigstens 5 mm aufweisen, zwischen denen weitgehend orthogonal zur Längserstreckung des bissschienenartig ausgebildeten Elements (8) die wenigstens eine Durchgangsöffnung (15) kanalartig verläuft.

5. Antischnarchvorrichtung nach Anspruch 4,
**dadurch gekennzeichnet, dass** der Verdrängungskörper (9) über den Verbindungsabschnitt (14) mit der konkaven Seite des bissschienenartig ausgebildeten Elementes (8) mit diesem verbunden ist.

6. Antischnarchvorrichtung nach Anspruch 4 oder 5,
**dadurch gekennzeichnet, dass** der kugelförmige Verdrängungskörper (9) oder die kugelartigen Elemente (91, 92) jeweils einen Kugelmittelpunkt (M) aufweisen,
dass der Kugelmittelpunkt (M) des kugelförmigen Verdrängungskörpers (9) oder die Kugelmittelpunkte (M) beider kugelartiger Elemente (91, 92) in Projektion parallel zu wenigstens einer Bissfläche (10, 11) außerhalb einer zwischen beiden Bissflächen weitgehend gleich beabstandeten Mittenebene (A) liegt.

7. Antischnarchvorrichtung nach Anspruch 6,
**dadurch gekennzeichnet, dass** der Kugelmittelpunkt (M) oder die Kugelmittelpunkte (M) in Projektion zu wenigstens einer Bissfläche (10, 11) näher zur unteren Bissfläche (11) als zur oberen Bissfläche (10) liegt bzw. liegen.

8. Antischnarchvorrichtung nach einem der Ansprüche 4 bis 7,
**dadurch gekennzeichnet, dass** die obere und untere Bissfläche (10, 11) längs zur gekrümmten Längserstreckung des bissschienenartig ausgebildeten Elementes (8) jeweils eine Begrenzungskante (12, 13) vorsehen, gegen die jeweils die Vorderseiten der Zähne des Ober- und Unterkiefers (3, 7) anstoßen.

9. Antischnarchvorrichtung nach einem der Ansprüche 4 bis 8,
**dadurch gekennzeichnet, dass** das bissschienenartig ausgebildete Element (8) eine gekrümmte Länge von 3 bis 4 cm aufweist, und
dass die wenigstens eine Durchgangsöffnung (15) einen Öffnungsdurchmesser von 2,5 bis 3,5 mm aufweist.

10. Antischnarchvorrichtung nach einem der Ansprüche 1 bis 9,
**dadurch gekennzeichnet, dass** das bissschienenartig ausgebildete Element (8) oder der Verdrängungskörper (9) über eine verstellbare Zugvorrichtung, die an den Ohren gehalten wird, verbunden ist, so dass die Zunge im Schlaf zum Gaumen hin gedrückt wird,

11. Antischnarchvorrichtung nach Anspruch 10,
**dadurch gekennzeichnet, dass** die Zugvorrichtung aus einem schnurähnlichen, flexiblen Material besteht und Mittel zur Längenveränderung aufweist.

## Claims

1. An anti-snoring device for positioning within the mouth cavity of a person, comprising a displacement member (9), of which the shape and size allow placement within the mouth cavity (4) between the bottom of the tongue and the musculature at the bottom of the mouth and which lifts the tongue (2) in toward the mouth cavity, the displacement member (9) cooperating with a mouthpiece-shaped element (8) that can be placed between the teeth of the upper and lower jaw (7, 3) and that provides at least one through-hole (15) for ventilation of the mouth cavity with breathed air, **characterised in that** the displacement member (9) is spherical, or **in that** the displacement member (9) comprises two uniformly spherical elements (91, 92), of which the ball shapes penetrate one another locally in part.

2. The anti-snoring device according to claim 1, **characterised in that** the spherical displacement member (9) or the spherical elements (91, 92) each have a diameter between 1.5 and 2.0 cm.

3. The anti-snoring device according to claim 1 or 2, **characterised in that** the displacement member (9) and the mouthpiece-shaped element (8) are rigidly connected via a connection portion (14).

4. The anti-snoring device according to one of claims 1 to 3, **characterised in that** the mouthpiece-shaped element (8) is longitudinally curved, with an upper and lower bite surface (10, 11) that have a distance of at least 5 mm oriented orthogonally to the two bite surfaces, between which the at least one through-hole (15) extends in a channel-like manner largely orthogonally to the longitudinal extension of the mouthpiece-shaped element (8).

5. The anti-snoring device according to claim 4, **characterised in that** the displacement member (9) is connected, via the connection portion (14) with the concave side of the mouthpiece-shaped element (8), to this element.

6. The anti-snoring device according to claim 4 or 5, **characterised in that** the spherical displacement member (9) or the spherical elements (91, 92) each comprise a ball centre point (M), **in that** the ball centre point (M) of the spherical displacement member (9) or the ball centre points (M) of both spherical elements (91, 92) project(s) parallel to at least one bite surface (10, 11) outside a central plane (A) distanced largely equally between the two bite surfaces.

7. The anti-snoring device according to claim 6, **characterised in that** the ball centre point (M) or the ball centre points (M) projecting towards at least one bite surface (10, 11) is/are arranged closer to the lower bite surface (11) than to the upper bite surface (10).

8. The anti-snoring device according to one of claims 4 to 7, **characterised in that** the upper and lower bite surfaces (10, 11) each provide a defining edge (12, 13) longitudinally to the curved longitudinal extension of the mouthpiece-shaped element (8), the front faces of the teeth of the upper and lower jaw (3, 7) contacting against these defining edges.

9. The anti-snoring device according to one of claims 4 to 8, **characterised in that** the mouthpiece-shaped element (8) has a curved length of 3 to 4 cm, and **in that** the at least one through-hole (15) comprises an opening diameter of 2.5 to 3.5 mm.

10. The anti-snoring device according to one of claims 1 to 9, **characterised in that** the mouthpiece-shaped element (8) or the displacement member (9) is connected via an adjustable tensioning device that is retained at the ears, in such a way that the tongue is pressed in toward the palate during sleep.

11. The anti-snoring device according to claim 10, **characterised in that** the tensioning device consists of a band-like, flexible material and comprises means for adjusting its length.

## Revendications

1. Dispositif anti-ronflement destiné à être positionné à l'intérieur de la cavité buccale d'une personne, comprenant un corps de déplacement (9) dont la forme et la taille permettent un placement à l'intérieur de la cavité buccale (4) entre la face inférieure de la langue et la musculature du plancher de la bouche, et soulève la langue (2) en direction de la cavité buccale, sachant que le corps de déplacement (9) est en liaison active avec un élément (8) de type rail à mordre, qui peut être placé entre les dents de la mâchoire supérieure et de la mâchoire inférieure (7, 3) et prévoit au moins une ouverture de passage (15) pour ventiler la cavité buccale avec de l'air, **caractérisé en ce que** le corps de déplacement (9) est conçu sphérique ou que le corps de déplacement (9) présente deux éléments (91, 92) conçus avec une même forme de type sphère, dont les formes sphériques se pénètrent partiellement localement.

2. Dispositif anti-ronflement selon la revendication 1, **caractérisé en ce que** le corps de déplacement sphérique (9) ou les éléments de type sphère (91, 92) possèdent respectivement un diamètre entre 1,5 et 2,0 cm.

3. Dispositif anti-ronflement selon la revendication 1 ou 2, **caractérisé en ce que** le corps de déplacement sphérique (9) et l'élément (8) de type rail à mordre sont reliés fixement par une section de liaison (14).

4. Dispositif anti-ronflement selon l'une des revendications 1 à 3, **caractérisé en ce que** l'élément (8) de type rail à mordre est incurvé longitudinalement, avec une surface à mordre (10, 11) supérieure et inférieure, lesquelles présentent un écart d'au moins 5 mm orienté de manière orthogonale aux deux surfaces à mordre, entre lesquelles l'au moins une ouverture de passage (15) s'étend en forme de canal de manière largement orthogonale par rapport à l'étirement en longueur de l'élément (8) de type rail à mordre.

5. Dispositif anti-ronflement selon la revendication 4, **caractérisé en ce que** le corps de déplacement (9) est relié à l'élément (8) de type rail à mordre avec le côté concave de celui-ci au-dessus de la section de liaison (14).

6. Dispositif anti-ronflement selon la revendication 4 ou 5, **caractérisé en ce que** le corps de déplacement sphérique (9) ou les éléments de type sphère (91, 92) présentent respectivement un point central de sphère (M), que le point central de sphère (M) du corps de déplacement sphérique (9) ou les points centraux de sphère (M) des deux éléments de type sphère (91, 92) repose (nt) en projection parallèlement à au moins une surface à mordre (10, 11) hors d'un plan médian (A) largement équidistant entre les deux surfaces à mordre.

7. Dispositif anti-ronflement selon la revendication 8, **caractérisé en ce que** le point central de sphère (M) ou les points centraux de sphère (M) repose(nt), en projection à au moins une surface à mordre (10, 11), plus près de la surface à mordre inférieure (11) que de la surface à mordre supérieure (10).

8. Dispositif anti-ronflement selon l'une des revendications 4 à 7, **caractérisé en ce que** les surfaces à mordre supérieure et inférieure (10, 11) prévoient respectivement une arête de délimitation (12, 13) longitudinalement par rapport à l'étirement longitudinal incurvé de l'élément (8) de type rail à mordre, contre laquelle respectivement les faces avant des dents des mâchoires supérieure et inférieure (3, 7) viennent buter.

9. Dispositif anti-ronflement selon l'une des revendications 4 à 8, **caractérisé en ce que** l'élément (8) de type rail à mordre présente une longueur incurvée de 3 à 4 cm, et que l'au moins une ouverture de passage (15) présente un diamètre d'ouverture de 2,5 à 3,5 mm.

10. Dispositif anti-ronflement selon l'une des revendications 1 à 9, **caractérisé en ce que** l'élément (8) de type rail à mordre ou le corps de déplacement (9) est maintenu par un dispositif de traction réglable tenu sur les oreilles, de façon à ce que la langue soit appuyée contre le palais pendant le sommeil.

11. Dispositif anti-ronflement selon la revendication 10, **caractérisé en ce que** le dispositif de traction est composé d'un matériau flexible semblable à un cordon et présente des moyens pour modifier la longueur.
